# EUROPEAN PATENT SPECIFICATION

(11) **EP 2 929 894 B1**
(45) Date of publication and mention of the grant of the patent: **21.02.2018**
(21) Application number: 13863256.7
(22) Date of filing: 09.12.2013
(51) Int. Cl.: A61K 39/39, A61K 39/08, A61K 39/09

(54) **VACCINATION ADJUVANT, AND PREPARATION AND VACCINES CONTAINING SAME**
IMPFUNGSADJUVANS, HERSTELLUNG UND IMPFSTOFFE DAMIT
ADJUVANT DE VACCINATION, PRÉPARATION ET VACCINS QUI LE CONTIENNENT

(30) Priority: 10.12.2012 UY 34506
(43) Date of publication of application: 14.10.2015
(73) Proprietor: Universidad de la Republica de Uruguay, Montevideo (UY)
(72) Inventor: FERREIRA CHIESA, Fernando Amaury, C.P. 11600 Montevideo (UY); QUIRICI VALADÁN, Leonora, C.P. 30000 Minas (UY); BESSIO PRATO, María Inés, C.P. 11400 Montevideo (UY); SILVEIRA GONZÁLEZ, Luis Fernando, C.P. 11200 Montevideo (UY)
(74) Representative: Durán-Corretjer, S.L.P.
(86) International application number: PCT/MX2013/000161
(87) International publication number: WO 2014/092528

(56) References cited:
- EP-A2- 0 533 492
- WO-A1-01/21151
- WO-A1-88/09336
- WO-A1-98/36772
- WO-A1-2010/042870
- WO-A2-02/074336
- WO-A2-02/074336
- PALATNIK-DE-SOUSA CLARISA B ET AL: "FML vaccine against canine visceral leishmaniasis: from second-generation to synthetic vaccine", EXPERT REVIEW OF VACCINES, EXPERT REVIEWS LTD, GB, vol. 7, no. 6, 1 August 2008 (2008-08-01), pages 833-851, XP009125352, ISSN: 1744-8395, DOI: 10.1586/14760584.7.6.833
- GAR CR CON NATHALIE ET AL: "Recent clinical experience with vaccines using MPL- and QS-21-containing adjuvant systems", EXPERT REVIEW OF VACCINES, EXPERT REVIEWS LTD, GB, vol. 10, no. 4, 1 April 2011 (2011-04-01), pages 471-486, XP009176124, ISSN: 1744-8395, DOI: 10.1586/ERV.11.29
- SILVEIRA F ET AL: "Quillaja brasiliensis saponins are less toxic than Quil A and have similar properties when used as an adjuvant for a viral antigen preparation.", VACCINE 15 NOV 2011, vol. 29, no. 49, 15 November 2011 (2011-11-15), pages 9177-9182, XP002746845, ISSN: 1873-2518
- BOMFORD R ET AL: "Adjuvanticity and ISCOM formation by structurally diverse saponins", VACCINE, ELSEVIER LTD, GB, vol. 10, no. 9, 1 January 1992 (1992-01-01), pages 572-577, XP023709410, ISSN: 0264-410X, DOI: 10.1016/0264-410X(92)90435-M [retrieved on 1992-01-01]
- VERZA, S.G. ET AL.: 'Micellar aggregates of saponins from Chenopodium quinoa: characterization by dinamic light scattering and transmission electron microscopy''.' DIE PHARMAZIE. vol. 67, no. 4, 01 April 2012, pages 288 - 292, XP055203268
- SIDHU, G.S. ET AL.: 'A mechanism for the hypocholesterolaemic activity of saponins''.' BRITISH JOURNAL OF NUTRITION vol. 55, no. 3, 01 May 1986, pages 643 - 649, XP055203269

## Description

### STATE OF THE ART

Many vaccines require the addition of components called adjuvants capable of enhancing the immune response against antigen(s). Adjuvants are a highly heterogeneous set of additives which can modulate and enhance the immune response against an antigen, allowing for the generation of a protective response generally using lower doses of antigen. The use of adjuvants has allowed the development of vaccine formulations with selective antigen stimulation, for modulating the immune response towards antibody production (humoral-type response) and/or cell-mediated (cellular-type response). The type of immune response induced by an adjuvant is usually a combination of both types of response, sometimes providing the prevalence of a response over another. Therefore, when designing vaccines is important to properly select the adjuvant to be employed in the formulation, in order to achieve an enhancement of the type of immune response required, toward one of the specific types of humoral or cellular response, or towards a combination of both.

Adjuvants generally are products that have the ability to directly stimulate the response of immune system cells to an antigen, or products that can act as carriers, allowing a more efficient antigen presentation to immune cells, which in turn leads to a better response. However, some adjuvants capable to act simultaneously as immunostimulants and antigen carriers have been described.

Despite its importance, few adjuvants are currently in use both in human and veterinary medicine. In particular, aluminum hydroxide or alumina is one of the few adjuvants approved by the FDA to be used in vaccine formulation for human use. It has physical properties that promote antigen binding through stable electrostatic attraction and forms suspensions that promote phagocytosis of antigen-presenting cells. Its mechanism of action has been recently described and is characterized by a response bias to a Th2-type profile with secretion of IL-4 and IL-5 cytokines, production of IgG1, as well as IgE and secretory IgA isotype immunoglobulin and activation of eosinophils and mast cells. Moreover, the stimulation of Th2 cell population exerts an antagonistic effect on Th1 cells inhibiting cell differentiation, so that alumina would not be the suitable adjuvant to combat intracellular pathogens (Lambrecht et al, 2009; Petrilli et al., 2007; Brewer et al., 1999). The vaccines formulated with alumina also have some practical problems as the preparations thereof are heterogeneous, produce granuloma at the site of injection and may become ineffective when frozen, which is a serious problem for conservation, mainly in third world countries (Lindblad, 2004).

Various products have been shown to have adjuvant activity, including mineral salts such as aluminum hydroxide or aluminum phosphates, liposomes, natural products including saponins and many others. In particular, some saponins from plants have proven to be very effective as adjuvants. Saponins are triterpene and steroid glycosides widely distributed in the plant kingdom. Due to their structural characteristics are amphiphilic surfactants, which explains its surfactant properties, ability to form colloidal solutions, hemolytic activity and ability to form mixed micelles with lipids and sterols. Saponins most studied and used as adjuvants are those from Chilean tree *Quillaja saponaria,* which have cellular and humoral adjuvant activity, and have been traditionally used in veterinary medicine (i.e., formulation of vaccines against foot-and-mouth disease) as well as saponins from other plant species. Saponins extracts from *Quillaja saponaria* with adjuvant activity are known and employed in commercial or experimental vaccines formulation, particularly saponin preparation called Quil A (Darslgaard, 1974). Also it has been described the isolation of pure saponins or better defined mixtures from Quil A product, which remain having adjuvant activity and lower toxicity than Quil A, for example a saponin called QS-21, which has been used or is being studied as an adjuvant for various types of vaccines (EP 0 362 279 B1 and US Pat. No. 5,057,540). Kensil also reported the isolation and adjuvant activity of others isolated Quil A saponins, including those called QS-7, 17, 18 and 21, being the last one the most studied and used as adjuvant.

Quil A saponins or purified saponins such as QS-21, either in solution or formulated in mixed micelles (ISCOMs or ISCOMATRIX®), promote strong immune responses (humoral and cellular responses with CLT activation) against a great variety of antigens. Quil A bias the response towards a Th1 profile associated with the production of the INF-y and IL-2 cytokines, stimulates production of high levels of total IgG and IgG1, IgG2a, IgG2b, IgG3 isotypes antibodies, provide robust DTH responses and cellular activation of CD8+ lymphocytes. Although Quil A is used in veterinary vaccines, their use in vaccines for human use has been restricted by its intense hemolytic activity and production of unwanted local reactions (fever, induration, tenderness, pain) at the injection site (Sun et al., 2009;. Kensil et al., 1991).

It has been shown that some colloidal systems based on saponins when co-administered with various antigens have an intense adjuvant activity, immune response modulation ability, and reduced side effects.

Currently, there is a growing interest in the development of colloidal formulations as antigen delivery systems. The liposomes, for example, allow for the encapsulation of proteins and antigenic peptides in a multimeric particulate form. However, due to lack of sufficient immunogenicity, these formulations generally require the use of further adjuvants to make them effective in stimulating an appropriate immune response.

Immunostimulatory complexes called ISCOMs are particulate antigen delivery systems comprising antigen, cholesterol, phospholipid and saponin (Quil A or other saponin) with potent immunostimulatory activity. ISCOMATRIX® is called the particulate adjuvant comprising cholesterol, phospholipids and saponins (Quil A) but without containing antigen. This adjuvant has essentially the same structure as ISCOMs, consisting of perforated cage-like particles of approximately 40 nm in diameter. The antigens can be formulated with ISCOMATRIX® to produce vaccines capable of antigen presentation and immunostimulants similar to ISCOMs-type formulations, but with a wider range of applicability, since its use is not limited to hydrophobic membrane proteins. Modifications of ISCOMs formulations and ISCOMATRIX® have also been developed to achieve a better association of some antigens, such as described in WO 98/36772.

ISCOMs and ISCOMATRIX®, combine the advantages of a particulate antigen delivery system with the *in situ* presence of an adjuvant (Quil A) and consequently have been found to be more immunogenic than other colloidal systems such as liposomes and protein micelles. Formulations of ISCOMs and ISCOMATRIX® retained the adjuvant activity of the Quil A, while increasing its stability, reducing its hemolytic activity, and producing less toxicity. They also generate a similar immune response to the one obtained by immunizing with simple mixtures of antigen and saponin, but allow for the use of substantially smaller amounts of antigen.

Several ISCOMs-type vaccine formulations or containing ISCOMATRIX® have been approved for veterinary use, for example against equine influenza virus. Other micellar systems mainly composed of saponins from *Q. saponaria* and sterols (primarily cholesterol) have been described, such as in WO 96/33739, being also formulated as emulsions such as described in US 2005/0220814 A1. To prepare such formulations different techniques to solubilize the most lipophilic components of the preparation are used, particularly to solubilize sterols, including use of detergents, previous dissolution in suitable organic solvents prior to mixing with aqueous solutions of saponins and/or antigens, formation of lipid films and their dissolution by saponins or detergents, and emulsification of lipid components prior to mixing with saponins. Obtaining these micellar formulations includes preparative steps due to low solubility of some ingredients in aqueous solutions and some of the steps also involve removing certain components, e.g., when detergents or organic solvents are used, according to the specific formulation process. Steps comprising purification of colloidal solutions prior to its use and vaccine formulation are also required in some preparations.

An alternative to the use of saponins from *Quillaja saponaria* Molina species is the use of saponins extracted from the *Quillaja brasiliensis* (A. St.-Hil. Et Tul.) Mart tree, native of southern Brazil and Uruguay, which saponins have proven to be effective as adjuvants, with a similar activity against viral antigens than Quil A (Silveira et al., Vaccine 29 (2011), 9177-9182). WO02/074336 discloses a vaccine comprising bile acid and a saponin.

### DESCRIPTION OF FIGURES

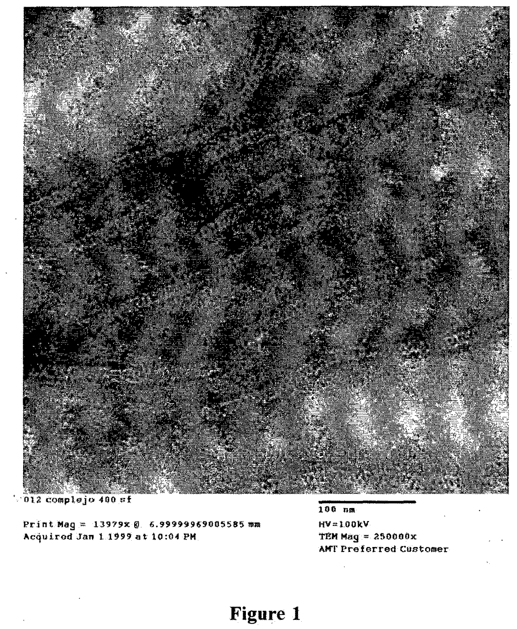

Figure 1: Image of Transmission Electron Microscopy (TEM) obtained by negative staining of micelles in a sample of colloidal solution comprising saponin and cholic acid prepared by mixing aqueous solutions of their components.

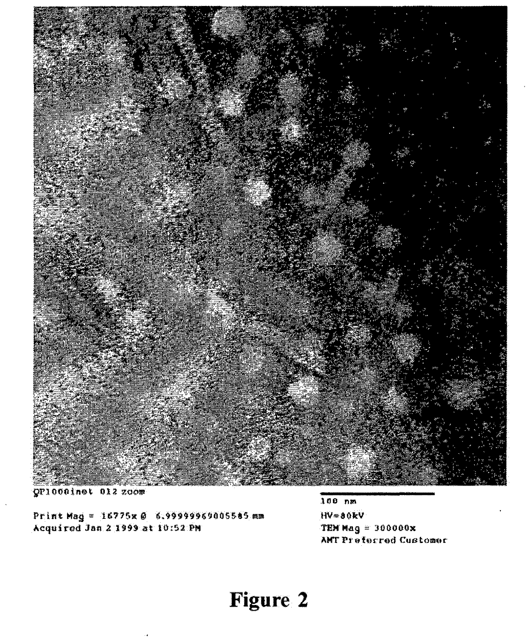

Figure 2: Image of Transmission Electron Microscopy (TEM) obtained by negative staining of micelles in a sample of colloidal solution comprising saponin and cholic acid prepared by the ethanol injection method.

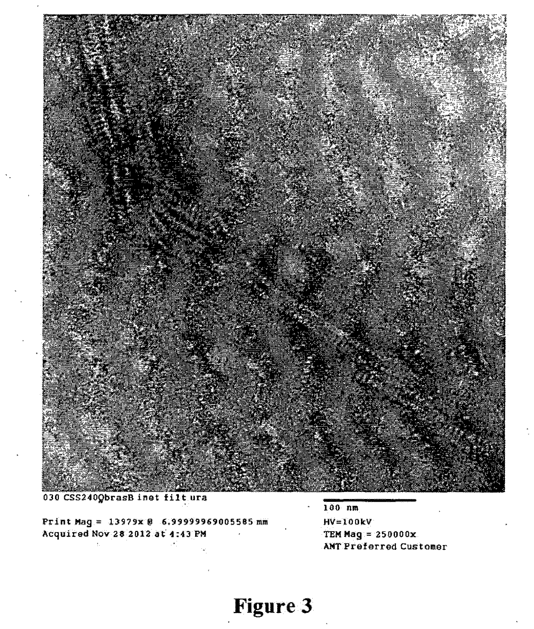

Figure 3: Image of Transmission Electron Microscopy (TEM) obtained by staining with uranyl acetate of micelles in a sample of colloidal solution comprising saponin from *Quillaja brasiliensis* and cholic acid prepared by the ethanol injection method.

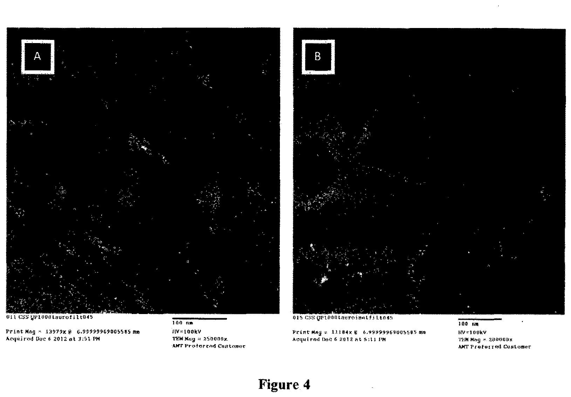

Figure 4: Image of Transmission Electron Microscopy (TEM) obtained by negative staining with uranyl acetate of micelles in: A) a sample of colloidal solution comprising saponins from *Quillaja saponaria* and sodium taurocholate prepared by mixing aqueous solutions of their components and B) a sample of colloidal solution comprising saponins from *Quillaja saponaria* and sodium taurocholate prepared by the ethanol injection method.

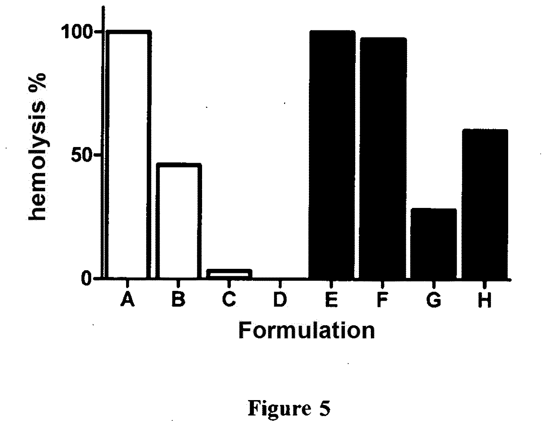

Figure 5: Hemolytic activity of different colloidal formulations comprising cholic acid and saponin.

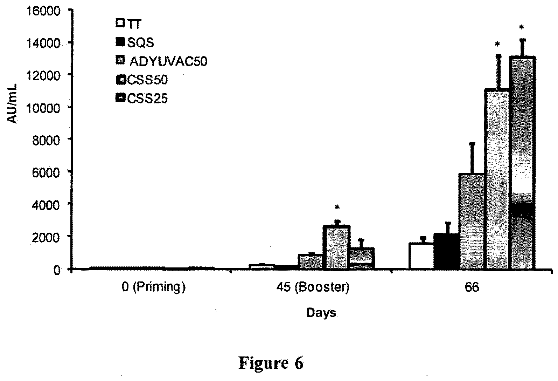

Figure 6: Titers of total IgG against tetanus toxoid in groups of mice immunized with tetanus toxoid alone in saline (TT), or with the following adjuvants: saponin (SQS), oil adjuvant (ADYUVAC50), adjuvant colloidal solution comprising acid colic and saponin (CSS25, 25 mg of saponin per dose) or adjuvant colloidal solution comprising cholic acid and saponin at higher concentration (CSS50, 50 mg of saponin per dose). Asterisk (*) is indicative of significant differences with TT group (P <0.05).

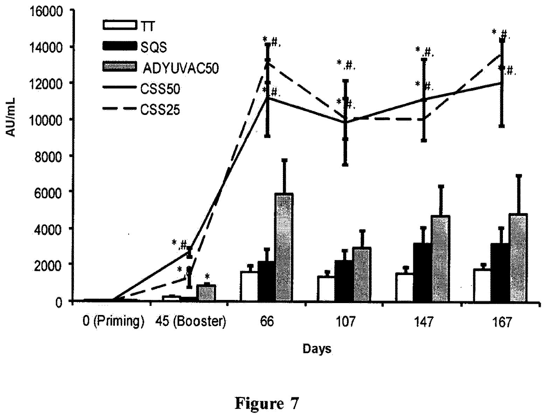

Figure 7: Change in time of total IgG titers against tetanus toxoid in groups of mice immunized with tetanus toxoid alone in saline (TT), or the following adjuvants: saponin (SQS), oil adjuvant (ADYUVAC50), adjuvant colloidal solution comprising cholic acid and saponin (CSS25, 25 µg saponin per dose) or adjuvant colloidal solution comprising cholic acid and saponin at higher concentration (CSS50, 50 µg of saponin per dose).

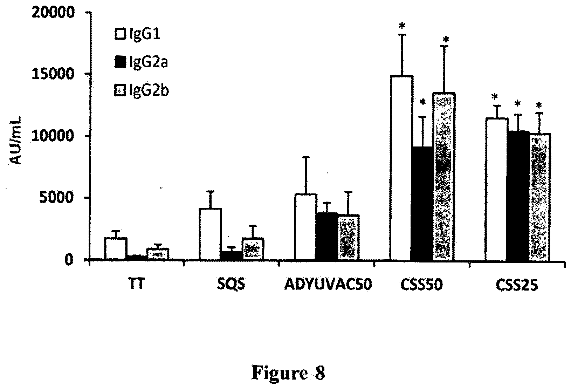

Figure 8: Titers of IgG isotypes against tetanus toxoid at day 107 of the experiment in groups of mice immunized with tetanus toxoid alone in saline (TT), or with the following adjuvants: saponin (SQS), oil adjuvant (ADYUVAC50), adjuvant colloidal solution comprising cholic acid and saponin (CSS25, 25 µg of saponin per dose) or adjuvant colloidal solution comprising cholic acid and saponin at higher concentration (CSS50, 50 µg of saponin per dose). Asterisk (*) indicates that titers of IgG1, IgG2a and IgG2b isotypes in CSS25 and CSS50 groups showed significant differences (P> 0.05) compared to the values found in all other animal groups included in the assay.

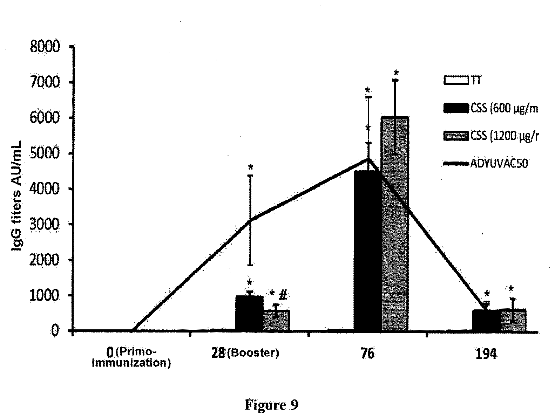

Figure 9. Change in time of total IgG titers against TT in sheep. Sheep were immunized on days 0 (primo-immunization) and 28 (booster) with TT alone or adjuvanted with ADYUVAC50, CSS600 or CSS1200. Data are presented as mean ± SE. Asterisk (*) is indicative of significant differences from the control group (TT only); and the symbol (#)is indicative of significant differences between CSS1200 and ADYUVAC50 groups.

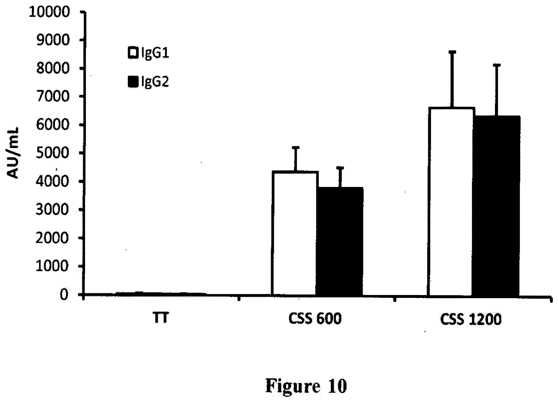

Figure 10: Titers of IgG1 and IgG2 subclasses against TT in sheep at day 76. TT: group immunized with tetanus toxoid in saline, CSS600: group immunized with tetanus toxoid co-administered with the adjuvant CSS600 colloidal solution, CSS1200: group immunized with toxoid tetanus co-administered with adjuvant CSS1200 colloidal solution. Titers are expressed in Arbitrary Units per mL (AU/mL).

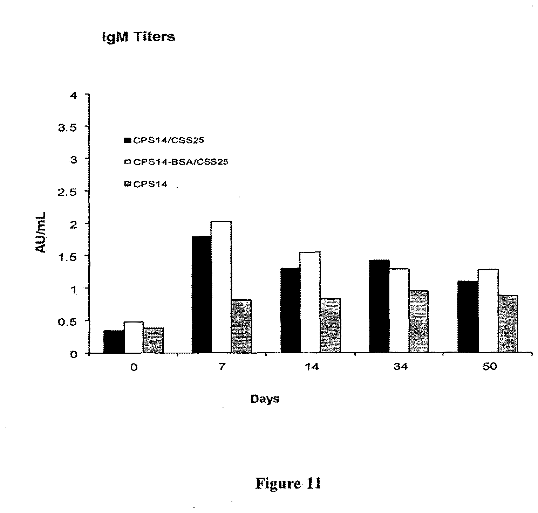

Figure 11: IgM titers specific against CPS14 in serum of mice immunized, on days 1 and 14, with CPS14 in one of the following vaccine preparations: CPS14 in saline (2.5 µg/dose, CPS14 group), CPS14 together with adjuvant colloidal solution comprising saponin and cholic acid (2.5 µg/dose, CPS14/CSS25 group), or the polysaccharide-protein conjugate CPS14-BSA together with adjuvant colloidal solution comprising saponin and cholic acid (1.8 µg/dose of carbohydrate, CPS14-BSA/CSS25 group).

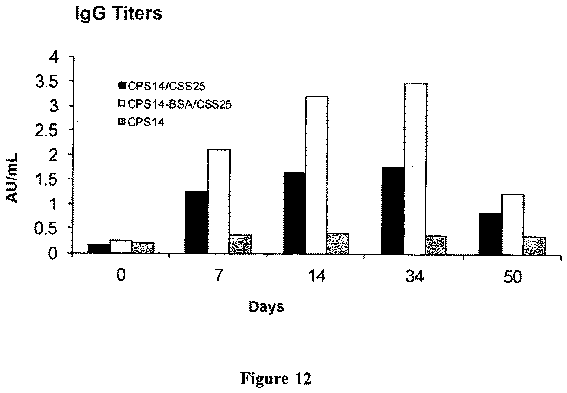

Figure 12: IgG titers specific against CPS14 in serum of mice immunized, on days 1 and 14, with CPS14 in one of the following vaccine preparations: CPS14 in saline (2.5 µg/dose CPS14 group), CPS14 together with the adjuvant colloidal solution comprising saponin and cholic acid (2.5 µg/dose, CPS14/CSS25 group), or the polysaccharide-protein conjugate CPS14-BSA together with adjuvant colloidal solution comprising saponin and cholic acid (1.8 µg/dose of carbohydrate, CPS14-BSA/CSS25 group).

### DESCRIPTION OF INVENTION

The present invention provides an adjuvant formulation comprising a saponin or saponin mixtures and one or more bile acids such as cholic acid or taurocholic acid, in definite proportions, as specified in the claims, giving rise to the formation of mixed micelles of both types of components, typically filamentous, and not necessarily requiring subsequent purification steps.

Also provided is a method for preparing a formulation, which does not require the use of additional components, since its preparation is possible by simple mixing dissolved components in water or buffer solutions which do not require their removal prior to use or for vaccine preparation. The formulation has the advantage that both end product and solutions from which the formulation is prepared, are aqueous solutions that can be sterilized by filtration, being possible, e.g., making the end product under aseptic conditions from component solutions previously sterilized by filtration. The end product can also be sterilized by filtration provided that antigen preparation is also sterilized by filtration.

Preparation process of the present invention essentially comprises the following steps: 1) making an aqueous solution of saponins to be used in the formulation, with or without a buffer or salt, 2) making a solution of appropriate concentration from bile acid solution or salts thereof to be used, preferably cholic acid, in a buffer solution of pH between 7.0 and 9.0, preferably between 7.5 and 8.5, preferably 8.0, 3) mixing both solutions so as to obtain relative ratios of bile acid to saponin between 2:1 and 5:1, preferably between 2.5:1 and 4:1 and more preferably 3:1, 4) adding an antigen solution, 5) pH correction to a range between 5.0-7.6, preferably between 6.5-7.5, if necessary, and performing a volume correction to bring to final concentrations for administration, and 6) ripening of mixture at temperatures preferably between 2°C and 8°C, preferably between 3°C and 5°C, preferably for a period between 12 and 96 hours, more preferably between 24 and 72 hours. According to the required concentrations of antigen and adjuvant in the end product for administration to animals or humans, a product dilution with a suitable solution is or is not performed.

Adjuvant solutions thus obtained essentially consists of a solution comprising saponin and bile acid, which can be associated or not with the antigen. Alternatively, antigen addition may be carried out after the formulation of adjuvant solution comprising saponin and bile acid.

Adjuvant solution described herein can be used as an adjuvant for different antigen types, including, but not limited to, viral antigens, proteins, carbohydrates and glycoconjugates, which can be defined or not, or could be complex antigenic preparations or antigen mixtures.

Both saponins, particularly saponins from *Quillaja saponaria,* Quil A and pure saponins isolated from Quil A as well as bile acids used in the preparation of the adjuvant formulation are surfactants, and therefore, above certain concentrations may occur lytic activity on cells, including red blood cells (hemolytic activity). However, the formulation object of the present invention, by combining the two components of the formulation in suitable ratios, can allow a substantial reduction of hemolytic activity of adjuvant formulation. Hemolytic activity of colloidal adjuvant formulation can be adjusted by varying the mass ratio of saponin and bile acid (cholic acid in particular or salts thereof), as well as varying total concentration of saponin in the formulation. In this way is possible to prepare adjuvant formulations containing concentrations of total saponins that are capable of producing 100% hemolysis under test conditions, which properly formulated may have reductions in said activity between 50% and almost 100%, that is, not having hemolytic activity under assay conditions. However, it is desirable, in some cases, that adjuvant formulations retain some lytic capacity, e.g., when formulations that improve or allow penetration of antigens through mucous membranes are sought, and in the present invention this is achievable by adjusting the relative ratios between saponin and bile acid.

Although the present invention uses a compounded adjuvant having by itself surfactant activity to prepare the formulation, both are part of the formulation, so the addition and subsequent removal of detergent is not required, which is the case with other adjuvant preparations in which the use of detergent is required to solubilize less water-soluble components. In turn, as additional steps of solvent removal are not required, e.g., dialysis, product manufacturing process is easier to scale-up to industrial level and is also simpler to produce according to good manufacturing practices (GMP).

The present invention does also not require the use of centrifugation steps in its preparation, as may occur in the case of formulations based on liposomes or ISCOMs, which in such cases could make production scale-up more difficult. The adjuvant object of the present invention can be manufactured directly from aqueous solutions of its components, unlike other adjuvants such as those described in US 6,506,386 B1, not requiring previous preparation of cholesterol containing liposomes for its production.

The colloidal adjuvant comprising saponins and bile acids object of the present invention is an aqueous colloidal solution which can be prepared by mixing sterile solutions of components thereof under aseptic conditions, which can be sterilized by filtration. This is an advantage relative to other adjuvants as alumina, which consists of suspensions that cannot be sterilized by filtration, or those adjuvants that may precipitate from solution after prolonged storage before use, making necessary stirring to achieve content uniformity inside the containers in which vaccines containing the adjuvant are dispense. Vaccines including aluminum salts as adjuvants also require to maintain a strict cold chain and vaccines can lose activity if they are subjected to excessively low temperatures that can cause freezing. The adjuvant formulations of the present invention are homogeneous aqueous colloidal solutions of low viscosity comprising saponin and bile acids that can be prepared in sterile form by filtration. This is an advantage over other adjuvants preparations consisting of emulsions of oil in water or water in oil, some of which cannot be filtered by sterilization, and can be difficult to administer parenterally because the high viscosity thereof, which can lead to incorrect dosing requiring the use of specialized personnel.

The present invention, because is related to homogeneous aqueous colloidal solutions of low viscosity, has advantages for human use compared to formulations containing aluminum salts as adjuvants, since aluminum salt being suspensions may frequently cause granulomas and pain at the injection site. For the same reasons, the present invention has advantages over adjuvant formulations that require the use of emulsions, some of which are highly viscous and produce local inflammation reactions and pain for which cannot be used in vaccines for human use.

However, it is also possible to prepare the adjuvant formulation object of the present invention using higher concentrations of saponin and bile acids and using the same directly in veterinary vaccines without causing significant local effects, for example using formulations containing up to 3.0 mg of saponins per mL or more, preferably between 0.120 mg/mL and 2.40 mg/mL of saponins, without causing appreciable local effects when administered parenterally to animals, for example sheep. The adjuvant object of the present invention can also be prepared at a higher concentration than the one that will be used in the formulation of the vaccine, such as 10 times more concentrated, and then be diluted to concentration of the final formulation using a suitable carrier such as, for example, PBS or isotonic saline.

The adjuvant object of the present invention can also be prepared by other methods, e.g., by the ethanol injection method. In this preparation method, the bile acid, e.g., cholic acid dissolved in ethanol, is added to an aqueous solution of saponin, for example at 0.240 mg/mL, in an alcohol volume not higher than 10% of the final volume, preferably not higher than 8% of the final volume, at such concentration that mass ratio between the bile acid or salts thereof and saponin in the final preparation is between 2 and 5, preferably between 2.5 and 4, more preferably 3. Alcohol used for formulation can be removed, e.g., by diafiltration, if required for formulation, or cannot be removed from the formulation if its presence does not cause undesirable side effects and does not affect vaccine activity such in the case of certain veterinary vaccines or for human use.

Saponins used in the present invention are preferably saponins from *Quillaja saponaria, Quillaja* brasiliensis, or Gypsophila tree, although saponins from other sources can be used. The present invention particularly includes adjuvant formulations comprising saponins from *Quillaja brasiliensis* and at least one bile acid, in which filamentous micelles are present, including a method of preparation thereof.

Preferably in the present invention preparations of saponins well known for their adjuvant activity such as Quil A, or isolated saponins, preferably QS-21, can be used, but the use of other fractions or preparations of saponins, particularly *Quillaja saponaria,* or other pure saponins is not excluded. In the present invention, and typically for veterinary use, saponins are used in concentrations between 60 µg/mL and 3,000 µg /mL, preferably in concentrations of 120 µg /mL and 2,400 µg /mL, more preferably between 240 µg /mL and 1,200 µg /mL, depending on the animal species, type and amount of antigen included in the vaccine and dosing volume administered. For human use, the formulation typically may contain an amount of saponin in a range of 1 µg to 100 µg per dose, preferably in a range of 10 µg to 50 µg per dose.

Adjuvant formulations comprised in the present invention contain a bile acid, typically cholic acid or another bile acid such as taurocholic acid among others, and a saponin in a mass ratio in the range of 2 to 5, preferably in a range of 2.5 to 4, more preferably in a ratio of 3. The adjuvant formulations included in the present invention may be prepared by mixing component solutions in water or buffer, as well as by solvent injection method in which bile acid in an ethanol solution is rapidly added to a saponin solution in water or buffer.

The present invention comprises vaccines or immunostimulants preparations comprising the adjuvant described herein together with an antigen or antigen mixture or antigen preparations, including preventive or therapeutic vaccines for human or animal use. The antigens may be formulated with the adjuvant described herein, may be well or not well defined antigens or antigen mixtures, including toxoids and bacterins and antigenic preparations containing viral or parasitic antigens. Vaccines described herein consist of adjuvant comprising saponin and one or more bile acids, preferably cholic acid, together with viral, bacterial or parasitic antigens as well as tumor antigens and chemically modified antigens including, but not limited to, protein antigens, polysaccharide antigens, carbohydrate antigens, glycoproteins and carbohydrate-protein conjugates, mucopolysaccharides, glycolipids, and other glycoconjugates and nucleic acids, both natural or modified and recombinant or synthetic, or antigen mixtures, such as in multivalent vaccines.

Antigens to be used in the vaccines comprised in the present invention preferably can be in aqueous solution or in aqueous salt solutions, or can also be suspensions or emulsions of antigenic preparations which can include preservatives, detergents, salts and other additives necessary for antigen preparation, together with the adjuvant comprising one or more bile acids, and saponins.

Colloidal adjuvant comprising saponin and bile acids described in the present invention has advantages over the use of saponin itself at the same concentration and over oil adjuvants, because it generates a more intense immune response and the antibody titers generated against co-administered antigen preparation are maintained in higher values for longer time, indicating that co-administration with an antigen preparation is capable of inducing a more effective and lasting immune response than the antigen alone or co-administered solely with saponin or an oil adjuvant.

Colloidal adjuvant comprising saponin and bile acids described herein may also be formulated in such a way that saponin concentration in the final formulation is sufficient to act as adjuvant in different antigenic preparations, allowing its adjustment to the antigen dose necessary to generate an effective immune response according to antigen type, and also the ratio of components that will reduce the hemolytic activity of the preparation and/or improve their adjuvant activity, within certain ranges, can be regulated.

Colloidal adjuvant comprising saponin and bile acids described in the present invention does not necessarily require the removal of any excess of any component that may be in relative excess, or which is not part of micelles, and can be used directly for the preparation of vaccine formulations, which represents an advantage in the production process and scale-up for its simplicity.

Colloidal adjuvant comprising saponin and bile acids described in the present invention has a very good ability to induce more intense immune responses against antigens co-administered with the same. This adjuvant containing saponin and bile acid is able to induce a more intense immune response against co-administered antigens of different nature, including, but not limited to, proteinaceous antigens, polysaccharides, protein-polysaccharide conjugates and other glycocompounds.

When co-administered with an antigen, colloidal adjuvant comprising saponin and bile acids described in the present invention generates a more intense immune response against said antigen that lasts longer after immunization, representing an advantage for generating a more lasting immune response, which may eventually allow the use of immunization schedules with more spaced immunizations compared to immunizations with an antigen or with other adjuvants such as saponin.

In this aspect, the colloidal adjuvant comprising saponin and bile acids described in the present invention is able to induce a more intense immune response for a given dose of antigen, which can eventually reduce the dose of antigen required to generate protective immune responses.

Colloidal adjuvant comprising saponin and bile acids described herein, which stimulates a more intense antigen immune response than administration of antigen alone, generates a balanced immune response because induces both a response of Th1 and Th2 type, which is an advantage in cases where it is necessary to induce a humoral and/or cellular response to generate protection against some pathogens such as viruses, intracellular pathogens and against tumor antigens in the case of preventive or therapeutic vaccines against tumor diseases.

The present invention is illustrated by the examples described below, but the present invention is not limited by them.

### EXAMPLES

EXAMPLE 1: Preparation of adjuvants solutions (supramolecular complexes, CSS, by its abbreviation in Spanish) comprising saponin and cholic acid or salts thereof by mixing aqueous solutions thereof.
Saponins to be used can have different origins, including *Quillaja saponaria, Quillaja brasiliensis, Gypsophila* sp., and other plant species as well as saponin preparations as Quil A or isolated saponins such as the one called QS-21. Cholic acid and salts thereof are well known natural compounds, and cholic acid or salts thereof of different purity, preferably greater than 90% purity, preferably greater than 98% can be used for making adjuvant formulation.
In this example the preparation of adjuvant colloidal solution composed of cholic acid or salts thereof and commercial saponin QP 1000 (Natural Response, Chile), or Quil A (Aquila Biopharmaceuticals, USA) is described.
A solution of 10 mg/mL cholic acid in a phosphate buffer pH 8.0 (solution A) and a 10 mg/mL aqueous solution of saponin from *Quillaja saponaria* (solution B) are made. To prepare 40 ml of formulation, 4.8 mL of solution A and 1.60 mL of solution B are mixed and 33.60 mL of isotonic saline is added, giving a mixture final pH about 7.5. The mixture is held between 24 and 72 hours at about 4°C before use. In Figure 1 is shown one of the images of Transmission Electron Microscopy (TEM) obtained for said formulation, mainly showing the formation of mixed filiform micelles of different lengths but with a relatively uniform diameter. These mixed micelles are formed at a saponin concentration below the critical micelle concentration of saponin, although higher concentrations of saponin can also be used. Using a diffusion experiment of passive dialysis, and quantifying the concentration of cholic acid in the outer and inner chambers of the dialysis bag it was observed a higher concentration in the inner chamber, showing that formation of mixed micelles of saponin and cholic acid inhibits passive diffusion of cholic acid through the dialysis membrane, which is consistent with formation of mixed micelles of both components.
EXAMPLE 2: Preparation of adjuvants solutions (supramolecular complexes, CSS) comprising saponin from *Quillaja saponaria* and cholic acid or salts thereof by the ethanol injection method.
This example shows an alternative method for making adjuvant colloidal solutions containing saponin and cholic acid using the ethanol injection method.
A 240 µg/mL saponin solution in TBS buffer pH = 7.4 is made, and 225 µL of an alcoholic solution of 9.6 mg/mL cholic acid are added to 2.775 mL of this saponin solution by rapid injection to give a colloidal solution with a mass ratio of saponin to cholic acid of 3 to 1. The solution is incubated at 4°C with stirring for 48 h before performing a transmission electron microscopy analysis. Samples were prepared for observation by negative staining using 2% phosphotungstic acid solution. Figure 2 shows an electron microscope image where the presence of filiform micelles of variable length and relatively uniform width is observed.
Zeta potential values determined by PCS (Photo Correlation Spectroscopy) for the formulations described in Example 1 and Example 2 were within the range of -14.0 mV to - 20.9 mV, showing that these formulations comprise micelles negatively charged.
EXAMPLE 3 Preparation of adjuvant colloidal solutions containing cholic acid and saponins from *Quillaja brasiliensis.*
In this example it is shown a method for the preparation of colloidal solutions of saponins from *Quillaja brasiliensis* and cholic acid using the ethanol injection method, showing that filiform mixed micelles can be formulated using saponins derived from other plant species.
For making this colloidal solution were employed the same experimental conditions detailed in Example 2, with the only difference that a 240 µg/mL solution of saponin from *Quillaja brasiliensis* was used. Saponins from *Quillaja brasiliensis* were obtained from an aqueous solution of leaves by solid phase extraction in reverse phase eluting with a gradient of methanol in water. Saponins were detected by thin-layer chromatography and compared to a Quil A standard. In Figure 3 is shown an image of transmission electron microscopy obtained using negative staining with uranyl acetate, wherein the presence of variable length and relatively uniform wide filiform micelles is observed.
EXAMPLE 4 Preparation of adjuvants colloidal solutions (supramolecular complexes, CSS) comprising saponins from *Quillaja saponaria* and taurocholic acid or salts thereof by the above mentioned methods.
In this example the preparation of colloidal solutions of saponins from *Quillaja saponaria* and sodium taurocholate is described, demonstrating that it is possible formulate filiform mixed micelles using other bile acid or salts thereof.
To prepare these colloidal solutions the following methods were used: a) method described in Example 1 b) method described in Example 2, and in both cases, the final concentrations of saponins from *Quillaja saponaria* and sodium taurocholate were 400 µg/mL and 1,200 µg/mL, respectively. Figure 4 shows images of transmission electron microscopy with negative staining using uranyl acetate: A) according to the method described in example 1 and B) according to the method described in example 2, in both cases the presence of variable length and relatively uniform wide filiform micelles is observed.
EXAMPLE 5: Preparation of adjuvant colloidal solutions containing saponin and cholic acid or salts thereof in various mass ratios (m/m) and its effect on reducing hemolytic activity of formulation.
It has been shown that saponins, especially saponins from *Quillaja saponaria,* have a strong adjuvant activity, but have the disadvantage of their ability to produce cell lysis, in particular red cell hemolysis, which can lead to undesirable side effects when administered parenterally. Hemolytic activity of saponins can be used as an estimate of its potential to produce undesirable local effects, so development of formulations containing saponins and simultaneously having a lower hemolytic activity has advantages for its use as adjuvants in immunogenic formulations and vaccines, in particular.
In the present example is shown how you can achieve micellar formulations of saponins from *Quillaja saponaria* and cholic acid with reduced hemolytic activity, as well as changing said hemolytic activity by varying the mass ratio between cholic acid (or salts thereof) and saponin.
For this example different formulations were prepared, varying mass ratios of cholic acid and saponins from *Quillaja saponaria.* For these formulations, a 10 mg/mL cholic acid solution in phosphate buffer pH 8.0 (solution A), and a 10 mg/mL aqueous solution of saponins of saponina QP UF 1000 (Natural Response, Chile) were used (solution B), and necessary amounts of isotonic saline to complete a 10 mL volume were added as detailed in Table 1.

**Table 1: Composition of formulations comprising cholic acid and saponin with different mass ratios (m/m) between both components**

| Formulation (mL) | Solution A (mL) | Solution B (mL) | Isotonic saline (mL) | cholic acid to saponins ratio (m/m) |
|---|---|---|---|---|
| A | 0 | 0.120 | 9.88 | 0 |
| B | 0.120 | 0.120 | 9.76 | 1 |
| c | 0.360 | 0.120 | 9.52 | 3 |
| D | 0.480 | 0.120 | 8.92 | 4 |
| E | 0 | 0.240 | 9.76 | 0 |
| F | 0.240 | 0.240 | 9.52 | 1 |
| G | 0.720 | 0.240 | 9.04 | 3 |
| H | 0.96 | 0.240 | 8.80 | 4 |

Hemolytic activity assay was conducted for different formulations as described in Silveira et al., Vaccine 29 (2011) 9177-9182. In Figure 5, hemolytic activity of formulations A to H is shown. Formulations A-D contain a final concentration of saponins of 0.120 mg/mL, and Formulations E-F contain a final concentration of saponins of 0.240 mg/mL. Formulation A is a solution of saponins alone at a concentration of 0.120 mg/mL, and Formulation E is a solution of saponins alone, at a concentration 0.240 mg/mL. Both Formulation A and E produced a 100% hemolysis according to the method used for its determination.
As shown in Figure 5, hemolytic activity of adjuvant colloidal formulations comprising colloidal cholic acid and saponin varies with the mass ratio of cholic acid to saponin. For formulation series with a final concentration of saponins of 0.120 mg/mL was found that hemolysis was 46%, 3% and 0% for formulations B, C and D containing a cholic acid to saponin ratio of 1, 3 and 4 (m/m), respectively. For formulation series with a final concentration of saponins of 0.240 mg/mL was found that hemolysis percentage was 97%, 28% and 60% for formulations F, G and H containing a cholic acid to saponin ratio of 1, 3 and 4, respectively.
This example demonstrates that hemolytic activity of formulations comprising cholic acid and saponin depends on saponin final concentration and mass ratio of saponin to cholic acid, so that it is possible, by adjusting the composition of the mixture, reduce or minimize saponin hemolytic activity, and therefore its potential for local side effects.
EXAMPLE 6: Formulation of vaccines for immunization of animals containing tetanus toxoid as antigen and colloidal solution of cholic acid and saponin as adjuvant.
In this example tetanus toxoid as antigen is used. The tetanus toxoid is used in a solution containing 3 mg/mL of protein equivalent to 450 Lf/mL (toxoid flocculation unit).
First, a 10 mg/mL cholic acid solution in a phosphate buffer pH 8.0 (solution A) and a 10 mg/mL aqueous solution of saponins QP UF 1000 (Natural Response, Chile) (solution B) are made. To prepare 40 mL of immunization formulation, typically 2.90 ml of solution A, 0.96 mL of solution B and 35.94 mL of normal saline are mixed. 0.2 mL of tetanus toxoid solution is added to that mixture, and the formulation is held for 12 to 96 hours at 4°C before use. All solutions used in this example are sterilized using 0.22 µm filter, and the formulation is prepared by mixing in a laminar flow chamber under aseptic conditions.
To prepare 40 mL of a formulation analogous to the previous one, but containing a saponin final concentration of 600 µg/mL (hereinafter CSS600) and 5 Lf of tetanus toxoid per mL, 7.20 ml of solution A, 2.40 mL of solution B, and 29.95 mL of isotonic saline are mixed. 0.45 mL of the tetanus toxoid solution mentioned in the above preparation are added to this mixture, formulation is held for 12 to 96 hours at 4°C before use. All solutions used in this example are sterilized using 0.22 µm filter, and the formulation is prepared by mixing under aseptic conditions.
To prepare 40 mL of a formulation analogous to the previous one, but containing a saponin final concentration of 1,200 µg/mL (hereinafter CSS1200) and 5 Lf of tetanus toxoid per mL, 14.4 ml of solution A, 4.80mL of solution B, and 20.35 mL of isotonic saline are mixed. 0.45 mL of the tetanus toxoid solution mentioned in the above preparation are added to this mixture, formulation is held for 24 to 72 hours in a refrigerator at 4°C before use. All solutions used in this example are sterilized using 0.22 µm filter, and the formulation is prepared by mixing in a laminar flow chamber under aseptic conditions.
EXAMPLE 7: Adjuvant effect of colloidal solution comprising cholic acid and saponin in a mouse model using tetanus toxoid (TT) as antigen, and comparison of adjuvant activity of said formulation with saponin and with an oil adjuvant.
For this example, adult male Balb/c mice (30 g) were used. The animals were adapted for 30 days prior to immunization assays with a day/night cycle of 12/12 hours, under controlled humidity and temperature. Water and pelleted food were provided *ad libitum.*
The tested mice groups received tetanus toxoid (TT) either alone or adjuvanted with saponin (QP UF 1000, Natural Response, Chile), or oil adjuvant ADYUVAC50 (Lab Santa Elena, Uruguay), or adjuvant colloidal solution comprising saponin and cholic acid, prepared as described in Example 6, but with a saponin final concentration of 500 µg/mL and a cholic acid to saponin mass ratio equal to 3. Mice (groups of 5 animals) received two immunizations with TT via SC route: the first on day 1 and the second on day 45. Primo-immunization was performed with 1.6 µg (100 µL) of TT except for CSS50 animal group which was immunized with 3.2 µg. Different groups received as adjuvant: saponin (QP UF 1000, Natural Response, Chile) (25 µg) or ADYUVAC50 (Laboratorios Santa Elena, prepared according to manufacturer's instructions) or adjuvant colloidal solutions of saponin and cholic acid prepared according to Example 6 (referred as CSS25 and containing 25 µg of saponin per dose) or adjuvant colloidal solutions of saponin and cholic acid prepared as above with a saponin final concentration of 500 µg/mL (referred as CSS50 and containing 50 µg of saponin per dose) in a final volume of 100 µL. The control group received only TT (1.6 µg in saline, 100 µL). The second dose (day 45) was formulated with half of antigen (0.8 µg) and adjuvant except for the ADYUVAC50 group wherein the antigen is reduced in half (0.8 µg), but the amount of adjuvant per dose was maintained. Animals were bled on days 1, 45, 66, 107, 147 and 167 after the first immunization and then sera were frozen at -20 °C until processing.
Titers of total IgG and IgG isotypes against TT in mice sera were assessed by ELISA. Plates (Greiner Bio-One) were sensitized with TT (100 µg/mL) in PBS (100 µL/well) overnight at 4 °C. After sensitization, the plates were washed three times with PBS containing 0.05% (v/v) Tween 20 (PBS/T) and blocked by incubating 1 hour at 37°C with a 5% (w/v) powdered bovine milk solution in PBS (200 µL/well). Then, plates were again washed as described above and incubated for 1 hour at 37°C with sera diluted in PBS/T containing 0.1% BSA (w/v) (PBS/T/BSA), seeded in duplicate (100 µL/well). After washing again, plates were incubated 1 hour at 37°C with appropriate dilutions of the corresponding peroxidase-conjugated antibody in PBS/T/BSA: anti-IgG (Jackson Immuno Research Laboratories, USA), IgG1 (Caltag Laboratories, USA), mouse IgG2a or IgG2b (Zymed Laboratories, USA) (100 µL/well). Finally, plates were washed and 100 µL per well of a solution containing substrate and chromogen (H2O2 + ABTS) was added; after 15 minutes OD at 405 nm was read. In each plate, serial dilutions of a sera pool (from the corresponding experiment) were included for generating a calibration curve which was used to express antibody titer in arbitrary units/mL (AU/mL).
Figure 6 shows total IgG antibody titers against tetanus toxoid (TT) in this assay measured at days 0, 45 and 66 in a mouse model immunized with antigen alone, or with antigen together with various adjuvants, this figure shows anti-TT IgG titers corresponding to the primary response and the response after a second administration of immunizing formulation. In this figure can be seen that co-administration of antigen with the adjuvant colloidal solution object of the present invention increases both primary and secondary immune responses in the murine model demonstrating that the adjuvant comprising saponin and cholic acid allows the generation of significantly higher antibody titers than the administration of antigen alone. This adjuvant colloidal solution comprising cholic acid and saponin is also more effective to stimulate a humoral immune response than saponin alone or commercial oil adjuvant ADYUVAC50, cause induce significantly higher titer secretion against the co-administered antigen.
Figure 7 shows change in time of total IgG titers against tetanus toxoid in different mice groups immunized with the various vaccine formulations described in this EXAMPLE. It is observed therein that adjuvant formulations containing colloidal cholic acid and saponin (CSS25 and CSS50 groups) induced production of significantly higher IgG titers against the co-administered antigen and remained at higher levels for longer time than IgG titers produced when the antigen is administered alone or together with saponin or with oil adjuvant ADYUVAC50. This result demonstrates that the colloidal adjuvant comprising saponin and cholic acid is effective to generate a more intense and lasting humoral response than the one generated by administration of antigen alone or together with saponin or formulated together with an oil adjuvant in the form of an emulsion .
Figure 8 shows titers of IgG1, IgG2a and IgG2b immunoglobulin subclasses in serum of different test animal groups immunized against TT. In general, it can be seen that the total production of IgG and isotypes analyzed (IgG1, IgG2a and IgG2b) against TT using formulations CSS25 and CSS50 had a similar behavior. Total IgG titers in CSS25 group were still higher than those in CSS50 group, although no statistically significant differences between them (P> 0.05) was found. The CSS adjuvant stimulated the production of higher titers of IgG1, IgG2a and IgG2b subclasses. This stimulation profile of all three immunoglobulin isotypes secretion has been described for ISCOMs type formulations in murine model (Sjolander et al. 1997), and is indicative of the generation of a balanced response between the Th1- and Th2-type response, suggesting stimulation of a response both humoral and cellular. CSS formulation also increased the duration of humoral response since titers of total IgG and isotypes remained still elevated almost six months after primo-immunization.
In summary, this EXAMPLE demonstrates that colloidal adjuvant formulation comprising cholic acid and saponin stimulates a more intense and lasting immune response when co-administered with tetanus toxoid than the one induced by antigen alone (tetanus toxoid), or when administered together with saponin as adjuvant, or formulated together with oil adjuvant ADYUVAC50, in which case the vaccine formulation is forming an emulsion. In this example generation of a balanced immune response is also shown, since a balanced increased of IgG1, IgG2a and IgG2b isotypes secretion against the co-administered antigen is achieved by using a solution comprising cholic acid and saponin as adjuvant, suggesting stimulation of an immune response both humoral and cellular, which is important for generating protective immune responses particularly against intracellular pathogens, viruses and against tumor antigens for cancer therapy.
EXAMPLE 8: Adjuvant Effect of colloidal solution of cholic acid and saponin in sheep using tetanus toxoid (TT) as antigen, and comparison of adjuvant activity of said formulation with an oil adjuvant.
For the assay of the present example, sheep were immunized with the vaccine formulations containing colloidal adjuvant comprising cholic acid and saponin and tetanus toxoid called CSS600 and CSS 1200, prepared as described in Example 6. These preparations have a cholic acid to saponin mass ratio equal to 3, and 5 Lf of tetanus toxoid per mL.
Sheep used, which are referred in this example, were kept under natural field conditions during the experiment and had no prior immunization against tetanus toxoid. Sheep were divided into 5 groups of 10 animals each and received two immunizations via SC route, on days 1 and 28 of the experiment. animals from different groups received 1 mL of various vaccine preparations, all containing the same amount of antigen equivalent to 5 Lf of tetanus toxoid per mL of vaccine. The vaccine formulations used were: saline tetanus toxoid (TT group), tetanus toxoid formulated with oil adjuvant ADYUVAC50 (Lab Santa Elena, Uruguay) used for preparation of the formulation according to the manufacturer's instructions (group ADYUVAC50), tetanus toxoid formulated with colloidal adjuvant containing cholic acid and saponin prepared as described in Example 6 and called CSS600 (CSS600 group) and tetanus toxoid formulated with colloidal adjuvant containing cholic acid and saponin prepared as described in Example 6 and called CSS1200 (CSS1200 group). Animals were bled on days 1, 28, 76 and 195 after the first immunization and sera were stored at -20 °C until processing.
Titers of total IgG and IgG isotypes against TT in sheep sera were assessed by ELISA. Plates (Greiner Bio-One) were sensitized with TT (100 µg/mL) in PBS (100 µL/well) overnight at 4 °C. After sensitization, the plates were washed three times with PBS containing 0.05% (v/v) Tween 20 (PBS/T) and blocked by incubating 1 hour at 37°C with a 5% (w/v) powdered bovine milk solution in PBS (200 µL/well). Then, plates were again washed and incubated for 1 hour at 37°C with sera diluted in PBS/T containing 0.1% BSA (w/v) (PBS/T/BSA), seeded in duplicate (100 µL/well). After washing again, plates were incubated with an antibody against total sheep IgG, or mouse monoclonal antibodies anti-sheep IgG1 or IgG2 overnight at 4°C. Then, plates were washed in the usual way and peroxidase-conjugated anti-mouse IgG antiserum with appropriate dilutions in PBS/T/BSA (Jackson Immuno Research Laboratories, USA) was added. Finally, plates were washed and a solution containing substrate and chromogen (H2O2 + ABTS) was added (100 µL per well); after 15 minutes OD at 405 nm was read. In each plate, serial dilutions of a sera pool (from the corresponding experiment) were included for generating a calibration curve which was used to express antibody titer in arbitrary units/mL (AU/mL).
Figure 9 shows total anti-TT IgG titers in sheep from different test groups.
All groups immunized with tetanus toxoid and an adjuvant (ADYUVAC50, CSS600 and CSS1200) generated higher anti-tetanus toxoid IgG titers than those generated in the group immunized with tetanus toxoid alone in saline. Upon primo-immunization higher antibody titers were observed in group ADYUVAC50 compared to CSS600 and CSS1200 groups, but after the second immunization, day 76 of experiment, CSS600 and CSS1200 animal groups showed anti-tetanus toxoid antibody titers equal or superior to those of animals of ADYUVAC50 group, and much higher than those animals immunized only with the antigen in saline (TT group). Figure 9 also shows that use of adjuvant colloidal formulations comprising cholic acid and saponin, called CSS600 and CSS1200 also generate high titers of antibodies against the co-administered antigen even 194 days after the first immunization, with statistically significant higher antibody titers compared to the group immunized without the use of adjuvant (TT Group). This experiment demonstrates that adjuvant colloidal formulations comprising cholic acid and saponin are as efficient as the oil adjuvant in the immunization scheme used for generating high antibody titers against the co-administered antigen and that said humoral immune response has a high duration in time.
Although ADYUVAC50 group had the highest total IgG titers after primo-immunization, oil adjuvants generally have the disadvantage of producing significant side effects (granuloma, erythema, abscess and swelling) at the site of inoculation (Aucouturier et al., 2006; Buonavoglia et al.; Gupta et al. January 93; Hilgers et al., 1994). In contrast, adjuvants comprising saponin and bile acids described in the present invention showed no appreciable side effects while promoting an intense humoral response. This aspect is of great economic importance since local side effects associated with immunization process not only cause pain and discomfort to animals, but have negative effects on meat and skins quality (Hilgers et al. 1994).
Titers of IgG1 and IgG2 immunoglobulins subclasses are shown in Figure 10. All groups immunized with TT and including adjuvants in the formulation gave measurable titers for both immunoglobulin subclasses. However, only groups immunized with tetanus toxoid including adjuvant formulations comprising cholic acid and saponin, referred as CSS600 and CSS1200 showed significant differences with control group (P <0.05), giving the highest IgG1 and IgG2 titers at day 76. It should be noted that titers of both IgG subclasses increased similarly, suggesting the ability of these adjuvants to produce a more intense immune response against the co-administered antigen and balanced between both types of response, at the same time.
It should be noted that the dose of saponins used by animal in the CSS formulations used in this example contained 0.6 to 1.2 mg of saponins, which are smaller doses than those used in other trials, however they generated an intense humoral immune response, with antibody titers similar to those obtained with oil adjuvant (ADYUVAC50).
EXAMPLE 9: Mice immunization assay using capsular polysaccharide antigen of *S. pneumoniae* serotype 14 (CPS14) or CPS conjugated with bovine serum albumin (BSA), together with the adjuvant formulations comprising cholic acid and saponin.
Antigens used for this example were: capsular polysaccharide of *S. pneumoniae* serotype 14 prepared as described in Suárez et al., Applied and Environmental Microbiology, 67 (2001) 969-971, and a conjugate of this polysaccharide with bovine serum albumin, referred as CPS14-BSA, prepared as described in Suárez et al., Journal of Chromatography -. A, 1213 (2008), 169-175.
For this example, adult male Balb/c mice (30 g) were used. Said animals were adapted for 30 days prior to immunization assays with a day/night cycle of 12/12 hours, under controlled humidity and temperature. Water and pelleted food were provided *ad libitum.*
Mice were divided into three groups of 8 animals each. Animals were immunized on day 1 (primo-immunization) and day 14 (booster) subcutaneously with 100 µL of vaccine preparation. The vaccine preparations used for immunization of different groups were: CPS14 saline with a concentration of 25 µg/mL (CPS14 control group), CPS14 solution in adjuvant colloidal solution comprising saponin and cholic acid prepared according to EXAMPLE 6 with a CPS14 concentration of 25 µg/mL (CPS14/CSS25 group), or a solution of polysaccharide-protein conjugate CPS14-BSA in adjuvant colloidal solution comprising saponin and cholic acid prepared according to EXAMPLE 6 with a concentration of carbohydrate in solution of 18.0 µg/mL (CPS14-BSA/CSS25 group). Animals were bled on days 1, 7, 14, 34 and 50 of experiment and sera were frozen at -20 °C until processing.
Titers of total IgG were evaluated as indicated in EXAMPLE 7, except that ELISA plates were coated with CPS14 (100 µg/mL) in PBS (100 µL/well) at 4 °C overnight. For determination of IgM titers same procedure was used, except by using appropriate dilutions of peroxidase-conjugated anti-mouse IgM antibody (Jackson Immuno Research Laboratories, USA) in PBS/T/BSA and analysis was conducted as described in EXAMPLE 6. IgM and IgG titers were expressed in arbitrary units by mL (AU/mL) using experiment pooled sera for generating a calibration curve. Figure 11 shows IgM titers for different groups of animals, which shows that co-administration of capsular polysaccharide CPS14 with adjuvant colloidal solution comprising saponin and cholic acid generated higher production of IgM titers specific to CPS14 than administration of CPS14 in saline. Figure 11 also shows that co-administration of CPS14-BSA with adjuvant solution comprising saponin and cholic acid generated higher production of IgM titers specific to CPS14 than administration of CPS14 in saline at day 7 and 14 after primo-immunization. At days 34 and 50, IgM titers specific to CPS14 obtained from the three groups of this example decreased to similar values.
Figure 12 shows IgG titers for different groups of animals in which it is observed that co-administration of capsular polysaccharide CPS14 with adjuvant colloidal solution comprising saponin and colic acid generated higher production of IgM titers specific to CPS14 than administration of CPS14 in saline. Figure 12 shows that while IgM titers specific to CPS14 remained almost constant at a low level throughout the experiment, group immunized with CPS14 together with adjuvant colloidal solution comprising saponin and cholic acid (group CPS14/CSS25) generated higher antibody titers that remained higher than the above group throughout the experiment. The results of this EXAMPLE shows that the adjuvant colloidal solution comprising saponin and cholic acid is effective for inducing a more intense humoral immune response against a polysaccharide antigen when is co-administered with the same, demonstrating the adjuvant colloidal solution is effective as a vaccination adjuvant for this type of antigens .
In the results of anti-CPS14 IgG titers in different groups of animals depicted in Figure 12 it is shown that titers of IgG antibodies specific to CPS14 in the group immunized with polysaccharide-protein conjugate together with the adjuvant colloidal solution comprising saponin and cholic acid (CPS14-BSA/CSS25 group) are higher than titers of the group immunized with CPS14 in saline (CPS14 group), and higher than titers of the group immunized with CPS14 together with the adjuvant colloidal solution comprising saponin and cholic acid (CPS14/CSS25 group). Such titers may also remained higher throughout the experiment with respect to other groups of experimental animals, suggesting that colloidal adjuvant comprising saponin and cholic acid may also have adjuvant activity when co-administered with antigens comprising polysaccharides or carbohydrates and proteins.

## Claims

1. An adjuvant preparation for vaccines comprising at least one bile acid or salts thereof and at least one saponin, **characterized in that** the relative mass ratio of bile acid or salts thereof to saponin is in a range between 2 and 5 (m/m), wherein said adjuvant preparation is an aqueous colloidal solution.

2. The adjuvant preparation according to claim 1, in which said relative mass ratio is in a range between 2.5 and 4 (m/m).

3. The adjuvant preparation according to claim 2, in which said relative mass ratio is equal to 3 (m/m).

4. The adjuvant preparation according to claims 1 to 3, in which said bile acid is cholic acid or salts thereof.

5. The adjuvant preparation according to claims 1 to 4, in which said bile acid is taurocholic acid or salts thereof.

6. The adjuvant preparation according to claims 1 to 5, in which said saponin is a preparation of saponins from *Quillaja saponaria* species.

7. The adjuvant preparation according to claims 1 to 6, in which said saponin is Quil A.

8. The adjuvant preparation according to claims 1 to 7, in which said saponin is QS-21.

9. The adjuvant preparation according to claims 1 to 8, in which said saponin is a saponin from *Quillaja brasiliensis* species.

10. The adjuvant preparation according to claims 1 to 9, in which said saponin is a saponin from *Gypsophila sp.*

11. The adjuvant preparation according to claims 1 to 10, in which said saponin concentration is in a range from 60 µg/mL to 3,000 µg/mL.

12. The adjuvant preparation according to claim 11, in which said saponin concentration is in a range from 60 µg/mL to 1,200 µg/mL.

13. The adjuvant preparation according to claims 1 to 12, in which said saponin concentration is in a range from 1 µg/mL to 100 µg/mL.

14. The adjuvant preparation according to claims 1 to 13, in which said saponin concentration is in a range from 10 µg/mL to 50 µg/mL.

15. The adjuvant preparation according to claim 1, in which said saponin is saponin preparation from *Quillaja saponaria* and the bile acid is cholic acid or salts thereof.

16. The adjuvant preparation according to claim 1, in which said saponin is Quil A and the bile acid is cholic acid or salts thereof.

17. A vaccine formulation comprising an adjuvant preparation as claimed according to anyone of claims 1 to 16 and one or more antigens including viral antigens, bacterial antigens, parasitic antigens, tumor antigens, protein antigens, polysaccharide antigens, carbohydrate antigens, glycoproteins, protein-carbohydrate conjugates, mucopolysaccharides, glycolipids, glycoconjugates, and both natural or modified, synthetic or recombinant nucleic acids, or antigen mixtures and antigen preparations.

18. A vaccine formulation as set forth in claim 17 comprising any antigen or antigenic preparation derived from *Clostridium, Streptococcus, Shigella, Salmonella, Neisseria, Borrelia, Bordetella, Plasmodium, Leptospira, Brucella, Haemophilus or Mycobacterium.*

19. The vaccine formulation as set forth in claim 17 comprising any antigen or antigenic preparation derived from Bovine Herpes Virus, Herpes Simplex Virus, Hepatitis A, B, C or E, or Rotavirus.

20. The vaccine formulation as set forth in claim 17 comprising an antigen comprising carbohydrates or an antigenic preparation derived thereof, including protein-polysaccharide conjugates and glycolipids.

21. The vaccine formulation as set forth in claim 17, in which the antigen comprises bacterial toxoids.

22. The vaccine formulation as set forth in claim 21, in which the antigen is tetanus toxoid.

23. A vaccine formulation as set forth in claim 17, in which the antigen comprises capsular polysaccharides of *Streptococcus pneumoniae.*

24. A vaccine formulation according to claim 23, in which the antigen is capsular polysaccharide of *Streptococcus pneumoniae* serotype 14.

25. A process for the production of an adjuvant preparation as claimed according to anyone of claims 1 to 16, comprising the following steps:
- preparing an aqueous solution of saponin or salts thereof, which may or may not contain a buffer;
- preparing an aqueous solution of bile acid or salts thereof, which may or may not contain a buffer;
- mixing the solutions prepared in a) and b) in the absence of additional detergent, in order to obtain a relative ratio of bile acid or salts thereof to saponin between 2 and 5 by mass;
- adjusting pH to a range between 5.0 and 7.6, if necessary; and
- held the mixture prepared in step c) at a temperature from 2°C to 12°C.

26. A process for the production of an adjuvant preparation as claimed according to anyone of claims 1 to 16, comprising the following steps:
- preparing an aqueous solution of saponin or salts thereof, which may or may not contain a buffer;
- preparing an ethanol solution of bile acid or salts thereof;
- rapid injection of the ethanol solution of bile acid into the aqueous solution of saponin, in order to obtain a relative ratio of bile acid to saponin between 2 and 5 by mass, and such a volume that no exceed 10% of the final volume of the preparation.

27. A process for manufacturing a vaccine formulation for human or animal use comprising adding an antigen or antigenic preparation at the time of mixing the components of adjuvant formulation prepared as described in step c) of claims 25 and 26.

28. A process for manufacturing a vaccine formulation for human or animal use comprising the following steps of:
- taking an adjuvant formulation prepared according to claims 26 and 27, and
- adding an antigen or antigenic preparation to this formulation.

29. The use of an adjuvant preparation according to anyone of claims 1 to 16 for formulating vaccines comprising antigens or antigenic preparations that require enhancement and modulation of the immune response against said antigens or antigenic preparations to generate an immune response effective in animals or humans.

## Patentansprüche

1. Adjuvanszubereitung für Vakzine, umfassend mindestens eine Gallensäure oder deren Salze und mindestens ein Saponin, **dadurch gekennzeichnet, dass** das relative Massenverhältnis von der Gallensäure oder deren Salzen zu dem Saponin in einem Bereich zwischen 2 und 5 (m/m) liegt, wobei die Adjuvanszubereitung eine wässrige, kolloidale Lösung ist.

2. Adjuvanszubereitung nach Anspruch 1, wobei das relative Massenverhältnis in einem Bereich zwischen 2,5 und 4 (m/m) liegt.

3. Adjuvanszubereitung nach Anspruch 2, wobei das relative Massenverhältnis gleich 3 (m/m) ist.

4. Adjuvanszubereitung nach den Ansprüchen 1 bis 3, wobei die Gallensäure Cholsäure oder deren Salze ist.

5. Adjuvanszubereitung nach den Ansprüchen 1 bis 4, wobei die Gallensäure Taurocholsäure oder deren Salze ist.

6. Adjuvanszubereitung nach den Ansprüchen 1 bis 5, wobei das Saponin eine Zubereitung aus Saponinen aus der Spezies *Quillaja saponaria* ist.

7. Adjuvanszubereitung nach den Ansprüchen 1 bis 6, wobei das Saponin Quil A ist.

8. Adjuvanszubereitung nach den Ansprüchen 1 bis 7, wobei das Saponin QS-21 ist.

9. Adjuvanszubereitung nach den Ansprüchen 1 bis 8, wobei das Saponin ein Saponin aus der Spezies *Quillaja brasiliensis* ist.

10. Adjuvanszubereitung nach den Ansprüchen 1 bis 9, wobei das Saponin ein Saponin aus *Gypsophila sp.* ist.

11. Adjuvanszubereitung nach den Ansprüchen 1 bis 10, wobei die Saponinkonzentration in einem Bereich von 60 µg/mL bis 3000 µg/mL liegt.

12. Adjuvanszubereitung nach Anspruch 11, wobei die Saponinkonzentration in einem Bereich von 60 µg/mL bis 1200 µg/mL liegt.

13. Adjuvanszubereitung nach den Ansprüchen 1 bis 12, wobei die Saponinkonzentration in einem Bereich von 1 µg/mL bis 100 µg/mL liegt.

14. Adjuvanszubereitung nach den Ansprüchen 1 bis 13, wobei die Saponinkonzentration in einem Bereich von 10 µg/mL bis 50 µg/mL liegt.

15. Adjuvanszubereitung nach Anspruch 1, wobei das Saponin eine Saponinzubereitung aus *Quillaja saponaria* und die Gallensäure Cholsäure oder deren Salze ist.

16. Adjuvanszubereitung nach Anspruch 1, wobei das Saponin Quil A und die Gallensäure Cholsäure oder deren Salze ist.

17. Vakzinformulierung, umfassend eine Adjuvanszubereitung nach einem der Ansprüche 1 bis 16 und ein oder mehrere Antigene, einschließlich von viralen Antigenen, bakteriellen Antigenen, parasitären Antigenen, Tumorantigenen, Proteinantigenen, Polysaccharidantigenen, Kohlenhydratantigenen, Glycoproteinen, Protein-Kohlenhydrat-Konjugaten, Mucopolysacchariden, Glycolipiden, Glycokonjugaten und sowohl natürlichen als auch modifizierten, synthetischen oder rekombinanten Nukleinsäuren oder Antigengemischungen und Antigenzubereitungen.

18. Vakzinformulierung nach Anspruch 17, umfassend irgendein Antigen oder antigene Zubereitung, abgeleitet von *Clostridium, Streptococcus, Shigella, Salmonella, Neisseria, Borrelia, Bordetella, Plasmodium, Leptospira, Brucella, Haemophilus* oder *Mycobacterium.*

19. Vakzinformulierung nach Anspruch 17, umfassend ein irgendein Antigen oder antigene Zubereitung, abgeleitet vom Rinderherpesvirus, Herpessimplex-Virus, Hepatitis A, B, C oder E oder Rotavirus.

20. Vakzinformulierung nach Anspruch 17, umfassend ein Antigen, das Kohlenhydrate umfasst, oder eine davon abgeleitete antigene Zubereitung, einschließlich von Protein-Polysaccharid-Konjugaten und Glycolipiden.

21. Vakzinformulierung nach Anspruch 17, wobei das Antigen bakterielle Toxoide umfasst.

22. Vakzinformulierung nach Anspruch 21, wobei das Antigen Tetanustoxoid ist.

23. Vakzinformulierung nach Anspruch 17, wobei das Antigen Kapselpolysaccharide von *Streptococcus pneumoniae* umfasst.

24. Vakzinformulierung nach Anspruch 23, wobei das Antigen Kapselpolysaccharide von *Streptococcus pneumoniae* Serotyp 14 umfasst.

25. Verfahren für die Herstellung von einer Adjuvanszubereitung nach einem der Ansprüche 1 bis 16, umfassend die folgenden Schritte:
- Herstellen von einer wässrigen Lösung von Saponin oder deren Salzen, die einen oder keinen Puffer enthalten kann;
- Herstellen von einer wässrigen Lösung von Gallensäure oder deren Salzen, die einen oder keinen Puffer enthalten kann;
- Mischen der Lösungen, die in a) und b) hergestellt werden, in Abwesenheit von zusätzlichem Detergens, um ein relatives Verhältnis von Gallensäure oder deren Salzen zu Saponin zwischen 2 und 5 bezogen auf die Masse zu erhalten;
- Einstellen von dem pH auf einem Bereich zwischen 5,0 und 7,6, falls erforderlich; und
- Halten von der Mischung, die in dem Schritt c) hergestellt wird, auf einer Temperatur von 2 °C bis 12 °C.

26. Verfahren für die Herstellung von einer Adjuvanszubereitung nach einem der Ansprüche 1 bis 16, umfassend die folgenden Schritte:
- Herstellen von einer wässrigen Lösung von Saponin oder deren Salzen, die einen oder keinen Puffer enthalten kann;
- Herstellen von einer Ethanollösung von Gallensäure oder deren Salzen;
- schnelles Injizieren von der Ethanollösung von Gallensäure in die wässrige Lösung von Saponin, um ein relatives Verhältnis von Gallensäure zu Saponin zwischen 2 und 5 bezogen auf die Masse zu erhalten, und zwar ein solches Volumen, das 10 % des Endvolumens von der Zubereitung nicht übersteigt.

27. Verfahren zum Herstellen von einer Vakzinformulierung zur Verwendung für Menschen oder Tiere, umfassend das Zugeben von einem Antigen oder einer antigenen Zubereitung zu dem Zeitpunkt des Mischens von den Komponenten der Adjuvansformulierung, die hergestellt wird, wie es in dem Schritt c) nach den Ansprüchen 25 und 26 beschrieben ist.

28. Verfahren zum Herstellen von einer Vakzinformulierung zur Verwendung für Menschen oder Tiere, umfassend die folgenden Schritte:
- Nehmen von einer Adjuvansformulierung, hergestellt nach den Ansprüchen 26 und 27, und
- Zugeben von einem Antigen oder einer antigenen Zubereitung zu dieser Formulierung.

29. Verwendung von einer Adjuvanszubereitung nach einem der Ansprüche 1 bis 16 zum Formulieren von Vakzinen, die Antigene oder antigene Zubereitungen umfassen, welche eine Steigerung und Modulation der Immunantwort gegen die Antigene oder antigenen Zubereitungen bedürfen, um eine Immunantwort zu erzeugen, die in Tieren oder Menschen wirksam ist.

## Revendications

1. Préparation d'adjuvant pour des vaccins, comprenant au moins un acide biliaire ou des sels de celui-ci et au moins une saponine, **caractérisée en ce que** le rapport de masse relative d'acide biliaire ou de sels de celui-ci vis-à-vis de la saponine est dans une plage comprise entre 2 et 5 (m/m), dans laquelle ladite préparation d'adjuvant est une solution colloïdale aqueuse.

2. Préparation d'adjuvant selon la revendication 1, dans laquelle ledit rapport de masse relative est dans une plage comprise entre 2.5 et 4 (m/m).

3. Préparation d'adjuvant selon la revendication 2, dans laquelle ledit rapport de masse relative est égal à 3 (m/m).

4. Préparation d'adjuvant selon les revendications 1 à 3, dans laquelle ledit acide biliaire est l'acide cholique ou des sels de celui-ci.

5. Préparation d'adjuvant selon les revendications 1 à 4, dans laquelle ledit acide biliaire est l'acide taurocholique ou des sels de celui-ci.

6. Préparation d'adjuvant selon les revendications 1 à 5, dans laquelle ladite saponine est une préparation de saponines à partir de l'espèce *Quillaja saponaria.*

7. Préparation d'adjuvant selon les revendications 1 à 6, dans laquelle ladite saponine est Quil A.

8. Préparation d'adjuvant selon les revendications 1 à 7, dans laquelle ladite saponine est QS-21.

9. Préparation d'adjuvant selon les revendications 1 à 8, dans laquelle ladite saponine est une saponine provenant de l'espèce *Quillaja brasiliensis.*

10. Préparation d'adjuvant selon les revendications 1 à 9, dans laquelle ladite saponine est une saponine provenant de l'espèce *Gypsophila.*

11. Préparation d'adjuvant selon les revendications 1 à 10, dans laquelle ladite concentration en saponine est dans une plage allant de 60 µg/mL à 3,000 µg/mL.

12. Préparation d'adjuvant selon la revendication 11, dans laquelle ladite concentration en saponine est dans une plage allant de 60 µg/mL à 1,200 µg/mL.

13. Préparation d'adjuvant selon les revendications 1 à 12, dans laquelle ladite concentration en saponine est dans une plage allant de 1 µg/mL à 100 µg/mL.

14. Préparation d'adjuvant selon les revendications 1 à 13, dans laquelle ladite concentration en saponine est dans une plage allant de 10 µg/mL à 50 µg/mL.

15. Préparation d'adjuvant selon la revendication 1, dans laquelle ladite saponine est une préparation de saponine à partir de l'espèce *Quillaja saponaria* et l'acide biliaire est l'acide cholique ou des sels de celui-ci.

16. Préparation d'adjuvant selon la revendication 1, dans laquelle ladite saponine est Quil A et l'acide biliaire est l'acide cholique ou des sels de celui-ci.

17. Formulation de vaccin comprenant une préparation d'adjuvant telle que revendiquée selon l'une quelconque des revendications 1 à 16 et un ou plusieurs antigènes incluant des antigènes viraux, des antigènes bactériens, des antigènes parasitaires, des antigènes tumoraux, des antigènes protéiques, des antigènes polysaccharides, des antigènes hydrates de carbone, des glycoprotéines, des conjugués protéine - hydrate de carbone, des mucopolysaccharides, des glycolipides, des glycoconjugués, et des acides nucléiques tant naturels que modifiés, synthétiques ou recombinants, ou des mélanges d'antigènes et des préparations d'antigènes.

18. Formulation de vaccin telle que définie dans la revendication 17, comprenant n'importe quelle préparation d'antigène ou antigénique dérivée de *Clostridium, Streptococcus, Shigella, Salmonella, Neisseria, Borrelia, Bordetella, Plasmodium, Leptospira, Brucella, Haemophilus ou Mycobacterium.*

19. Formulation de vaccin telle que définie dans la revendication 17, comprenant n'importe quelle préparation d'antigène ou antigénique dérivée du virus de l'herpès bovin, du virus de l'herpès simplex, du virus de l'hépatite A, B, C ou E, ou du rotavirus.

20. Formulation de vaccin telle que définie dans la revendication 17, comprenant un antigène comprenant des hydrates de carbone ou une préparation antigénique dérivée de celui-ci, incluant des conjugués protéine-polysaccharide et des glycolipides.

21. Formulation de vaccin telle que définie dans la revendication 17, dans laquelle l'antigène comprend des anatoxines bactériennes.

22. Formulation de vaccin telle que définie dans la revendication 21, dans laquelle l'antigène est l'anatoxine du tétanos.

23. Formulation de vaccin telle que définie dans la revendication 17, dans laquelle l'antigène comprend des polysaccharides capsulaires de *Streptococcus pneumoniae.*

24. Formulation de vaccin selon la revendication 23, dans laquelle l'antigène est le polysaccharide capsulaire du serotype 14 de *Streptococcus pneumoniae.*

25. Procédé pour produire une préparation d'adjuvant telle que revendiquée selon l'une quelconque des revendications 1 à 16, comprenant les étapes suivantes :
- préparer une solution aqueuse de saponine ou de sels de celle-ci, qui peut contenir ou ne pas contenir un tampon ;
- préparer une solution aqueuse d'acide biliaire ou de sels de celui-ci, qui peut contenir ou ne pas contenir un tampon ;
- mélanger les solutions préparées en a) et b) en l'absence de détergent additionnel, de façon à obtenir un rapport relatif de l'acide biliaire ou des sels de celui-ci vis-à-vis de la saponine compris entre 2 et 5 en masse ;
- ajuster le pH dans une plage comprise entre 5.0 et 7.6, si nécessaire ; et
- maintenir le mélange préparé dans l'étape c) à une température comprise entre 2 °C et 12°C.

26. Procédé pour produire une préparation d'adjuvant telle que revendiquée selon l'une quelconque des revendications 1 à 16, comprenant les étapes suivantes :
- préparer une solution aqueuse de saponine ou de sels de celle-ci, qui peut contenir ou ne pas contenir un tampon ;
- préparer une solution d'acide biliaire ou de sels de celui-ci dans l'éthanol ;
- injection rapide dans la solution aqueuse de saponine de la solution d'acide biliaire dans l'éthanol, de façon à obtenir un rapport relatif d'acide biliaire vis-à-vis de la saponine compris entre 2 et 5 en masse, et de façon qu'un tel volume n'excède pas 10 % du volume final de la préparation.

27. Procédé pour fabriquer une formulation de vaccin pour utilisation chez l'homme ou l'animal, comprenant l'ajout d'une préparation d'antigène ou antigénique au moment de mélanger les composants de la formulation d'adjuvant préparée comme décrit dans l'étape c) des revendications 25 et 26.

28. Procédé pour fabriquer une formulation de vaccin pour utilisation chez l'homme ou l'animal, comprenant les étapes suivantes de :
- prendre une formulation d'adjuvant préparée selon les revendications 26 et 27, et
- ajouter à cette formulation une préparation d'antigène ou antigénique.

29. Utilisation d'une préparation d'adjuvant selon l'une quelconque des revendications 1 à 16 pour formuler des vaccins comprenant des préparations d'antigènes ou antigéniques qui nécessitent l'augmentation et la modulation de la réponse immunitaire contre lesdites préparations d'antigènes ou antigéniques pour générer une réponse immunitaire efficace chez les animaux ou les humains.
